# EUROPÄISCHE PATENTSCHRIFT

(11) **EP 2 142 647 B1**
(45) Veröffentlichungstag und Bekanntmachung des Hinweises auf die Patenterteilung: **18.09.2013**
(21) Anmeldenummer: 08759386.9
(22) Anmeldetag: 25.04.2008
(51) Int. Cl.: C12N 11/04, C12Q 1/02

(54) **GANZZELLSENSOR**
WHOLE-CELL SENSOR
CAPTEUR ENTIÈREMENT CELLULAIRE

(30) Priorität: 25.04.2007 DE 102007020725
(43) Veröffentlichungstag der Anmeldung: 13.01.2010
(73) Patentinhaber: Technische Universität Dresden, 01062 Dresden (DE)
(72) Erfinder: OSTERMANN, Kai, 01187 Dresden (DE); POMPE, Wolfgang, 01337 Hartha (DE); BÖTTCHER, Horst, 01097 Dresden (DE); RÖDEL, Gerhard, 85757 Karlsfeld (DE); GROSS, Annett, 01069 Dresden (DE)
(74) Vertreter: Kailuweit & Uhlemann Patentanwälte
(86) Internationale Anmeldenummer: PCT/EP2008/055099
(87) Internationale Veröffentlichungsnummer: WO 2008/132178

(56) Entgegenhaltungen:
- WO-A-00/14267
- WO-A-02/21128
- WO-A-2005/069738
- WO-A-2007/145661
- DE-A1- 4 301 087
- DE-A1-102004 025 224
- GB-A- 2 364 307
- US-A- 6 117 643
- BELKIN S: "MICROBIAL WHOLE-CELL SENSING SYSTEMS OF ENVIRONMENTAL POLLUTANTS" CURRENT OPINION IN MICROBIOLOGY, CURRENT BIOLOGY LTD, GB, Bd. 6, Nr. 3, 1. Juni 2003 (2003-06-01), Seiten 206-212, XP001202139 ISSN: 1369-5274
- KWAN C.H.: "Biosensors for biological nutrient monitoring (Ph.D. thesis)." [Online] 2004, HONG KONG UNIVERSITY OF SCIENCE AND TECHNOLOGY , HONG KONG , XP002495849 Gefunden im Internet: URL:http://lbxml.ust.hk/th_imgo/b854858.pd f> [gefunden am 2008-09-10] Seite 3, Absatz 3 Seite 28, Absatz 2 - Seite 29, Absatz 2 Seite 60, Absatz 1 Seite 126, Absatz 2
- D'SOUZA S F: "Microbial biosensors" BIOSENSORS & BIOELECTRONICS ELSEVIER UK, Bd. 16, Nr. 6, August 2001 (2001-08), Seiten 337-353, XP002495847 ISSN: 0956-5663
- BARONIAN K H R: "The use of yeast and moulds as sensing elements in biosensors." BIOSENSORS & BIOELECTRONICS 15 APR 2004, Bd. 19, Nr. 9, 15. April 2004 (2004-04-15), Seiten 953-962, XP002495848 ISSN: 0956-5663
- SCHREITER P P -Y ET AL: "Monitoring of phosphorus bioavailability in water by an immobilized luminescent cyanobacterial reporter strain" BIOSENSORS & BIOELECTRONICS ELSEVIER UK, Bd. 16, Nr. 9-12, Dezember 2001 (2001-12), Seiten 811-818, XP009105537 ISSN: 0956-5663
- TAKAHASHI JUNKO ET AL: "Multiple reporter gene assays for the assessment and estimation of chemical toxicity" ENVIRONMENTAL SCIENCES: AN INTERNATIONAL JOURNAL ONENVIRONMENTAL PHYSIOLOGY AND TOXICOLOGY, TOKYO : SCIENT. PUBL. DIV. OF MYU, JP, Bd. 11, Nr. 5, 1. Januar 2004 (2004-01-01), Seiten 269-282, XP009096272 ISSN: 0915-955X
- PREMKUMAR J R ET AL: "SOL-GEL LUMINESCENCE BIOSENSORS: ENCAPSULATION OF RECOMBINANT E. COLI REPORTERS IN THICK SILICATE FILMS" ANALYTICA CHIMICA ACTA, ELSEVIER, AMSTERDAM, NL, Bd. 462, Nr. 1, 26. Juni 2002 (2002-06-26), Seiten 11-23, XP001202138 ISSN: 0003-2670
- BOER VIKTOR M ET AL: "The genome-wide transcriptional responses of Saccharomyces cerevisiae grown on glucose in aerobic chemostat cultures limited for carbon, nitrogen, phosphorus, or sulfur" JOURNAL OF BIOLOGICAL CHEMISTRY, AMERICAN SOCIETY OF BIOLOCHEMICAL BIOLOGISTS, BIRMINGHAM,; US, Bd. 278, Nr. 5, 31. Januar 2003 (2003-01-31), Seiten 3265-3274, XP002418951 ISSN: 0021-9258 in der Anmeldung erwähnt
- AKYILMAZ ET AL: "Sensitive determination of L-lysine with a new amperometric microbial biosensor based on Saccharomyces cerevisiae yeast cells" BIOSENSORS & BIOELECTRONICS, ELSEVIER SCIENCE PUBLISHERS, BARKING, GB, Bd. 22, Nr. 6, 14. Dezember 2006 (2006-12-14), Seiten 1055-1060, XP005730906 ISSN: 0956-5663
- ARMON R ET AL: "Sol-gel applications in environmental biotechnology", JOURNAL OF BIOTECHNOLOGY, ELSEVIER SCIENCE PUBLISHERS, AMSTERDAM, NL, vol. 51, no. 3, 15 November 1996 (1996-11-15), pages 279-285, XP004071573, ISSN: 0168-1656, DOI: 10.1016/S0168-1656(96)01607-0

## Beschreibung

Die Erfindung betrifft Ganzzellsensoren für das Monitoring von bioverfügbarem Stickstoff, Phosphor und/oder Schwefel in einem Medium und deren Verwendung.

Bisherige Lösungen für das Monitoring von bioverfügbarem Stickstoff, Phosphor und/oder Schwefel in einem wässrigen Medium beruhen auf
- dem Nachweis der Änderung der physikalischen oder chemischen Eigenschaften des Mediums mit einer Änderung der Konzentration des Analyten,
- auf selektiven Bindungsstudien der entsprechenden Ionen an geeigneten Membranen oder Cagestrukturen,
- sowie spezifischen Reaktionen der Analyten, in deren Ergebnis die Reaktionsprodukte detektiert werden können.

Daraus resultieren sehr verschiedenartig ausgestaltete Lösungen für elektrische, elektrochemische, optische oder colorimetrische Sensoren.

Bisher bekannten Lösungsvorschlägen ist gemeinsam, dass keiner von ihnen die direkte Wirkung von Stickstoff-, Phosphor- und/oder Schwefelverbindungen auf Prozesse in lebenden Zellen, also ihre biologische Verfügbarkeit, direkt zu bewerten gestattet. Hingegen ist es nur möglich, aus den gemessenen physikalischen oder chemischen Änderungen des Mediums indirekt auf mögliche Reaktionen in den im Medium lebenden Mikroorganismen zu schließen.

Boer et al. haben das gesamte Hefegenom auf Hefe-Gene hin untersucht, die als Antwort auf den Mangel von bioverfügbarem Stickstoff, Phosphor und/oder Schwefel aktiviert oder reprimiert werden. (Boer, V.M. et al. The genome-wider transcriptional responses of Saccharomyces cerevisiae grown on glucose in aerobic chemostat cultures limited for carbon, nitrogen, phosphorus, or sulfur. J. Biol. Chem. (2003) 278 (5) 3265-74). Die Expressionsprofile dienen als Indikator für die Charakterisierung von nährstofflimitierten Wachstumsbedingungen.

EP1426439A1 offenbart ein Verfahren zur Detektion von toxischen Substanzen. Dazu werden genetisch veränderte Hefezellen verwendet, wobei die Hefezellen ein Markergen unter der Kontrolle eines Promotors eines Gens enthalten, dessen Transkription sich in Anwesenheit der toxischen Substanz stark erhöht.

Inama et al. offenbaren Methoden zur Einbettung von lebenden Hefezellen in SiO₂-Sol-Gel-Schichten auf Glasträgern, zum Beispiel zur Verwendung als Biokatalysatoren (Inama, I. et al., Entrapment of viable microorganisms by SiO2 sol-gel layers on glass surfaces: trapping, catalytic performance and immobilization durability of Saccharomyces cerevisiae. J. Biotechnol. (1993) 30 (2) 197-210).

US 6 117 643 offenbart die Verwendung einzelliger Mikroorganismen als Bioreporter in einem monolithischen bioelektrischen Gerät.

Der im Patentanspruch 1 angegebenen Erfindung liegt die Aufgabe zugrunde, einen Biosensor zu entwickeln, mit dem einfach eine Limitierung von bioverfügbarem Stickstoff, bioverfügbarem Phosphor und/oder bioverfügbarem Schwefel in einem Medium nachgewiesen werden kann.

Diese Aufgabe wird mit den im Patentanspruch 1 aufgeführten Merkmalen gelöst.

Der Ganzzellsensor gemäß Anspruch 1 eignet sich zur Erfassung von bioverfügbarem Stickstoff, Phosphor und Schwefel jeweils einzeln oder in wenigstens einer Kombination in einem Medium. Er besteht aus gentechnisch veränderten Hefezellen, die in einer Xerogel-Matrix immobilisiert sind, wobei die Hefezellen mindestens ein Markergen unter der Kontrolle eines Promotors eines Gens enthalten, dessen Transkription sich bei Stickstoff-, Phosphor- oder Schwefel-Mangel stark erhöht oder stark erniedrigt, und wobei die Hefezellen wenigstens mit einem Signaldetektor gekoppelt sind.

Die Ganzzellsensoren für bioverfügbaren Stickstoff, Phosphor und Schwefel jeweils einzeln oder in wenigstens einer Kombination in einem Medium zeichnen sich insbesondere dadurch aus, dass eine Limitierung von bioverfügbarem Stickstoff, bioverfügbarem Phosphor und/oder bioverfügbarem Schwefel im Medium einfach nachzuweisen ist.

Die Medien, in denen der Nachweis erfolgt, sind vorzugsweise wässrige Medien. Dazu zählen beispielsweise Kulturmedien, die zur Heranzucht von Mikroorganismen wie Bakterien oder Hefen oder von Algen oder von tierischen und pflanzlichen Zellen verwendet werden. Ein großes Anwendungsgebiet des Biosensors sind Verfahren der Weißen Biotechnologie, wo Mikroorganismen zur Erzeugung von speziellen Produkten wie zum Beispiel spezieller chemischer Verbindungen mittels einer Biotransformation oder Biokatalyse zum Einsatz kommen. Verwendung finden die Biosensoren ebenfalls in Gärprozessen, bei denen Mikroorganismen zur Herstellung von Lebensmitteln eingesetzt werden, beispielsweise beim Brauen von Bier. Alternative Einsatzgebiete sind die Reinigung von Trinkwasser, industriellem Brauchwasser oder Abwasser, bei der der Gehalt an bioverfügbarem Stickstoff, Phosphor oder Schwefel bestimmt werden soll.

Lebende Zellen sind darauf angewiesen, ihren Bedarf an Stickstoff, Schwefel und Phosphor durch die Aufnahme von entsprechenden Stickstoff-, Schwefel- und Phosphor-haltigen Verbindungen zu decken. Die Verfügbarkeit solcher Verbindungen im Medium wird im Folgenden als bioverfügbarer Stickstoff, Schwefel oder Phosphor bezeichnet. Die erfindungsgemäßen Hefezellen ermöglichen im Gegensatz zu Messfühlern, die direkt den Stickstoff-, Phosphor- oder Schwefelgehalt des zu untersuchenden Mediums über physikalische oder physikochemische Eigenschaften des Mediums bestimmen, vorteilhaft die direkte Wirkung von Stickstoff, Phosphor- und/oder Schwefelverbindungen auf Prozesse in lebenden Zellen, also ihre biologische Verfügbarkeit, zu bewerten. Als Hefezellen werden bevorzugt *Saccharomyces cerevisiae* und *Schizosaccharomyces plombe* verwendet.

Dabei ist mindestens ein Markergen der Hefezellen unter die Kontrolle eines Promotors eines Gens gestellt, dessen Transkription sich bei Stickstoff-, Phosphor- oder Schwefel-Mangel stark erhöht oder stark erniedrigt. Unter stark erhöhter oder stark erniedrigter Transkription wird eine mindestens zweifache Erhöhung bzw. Erniedrigung der Transkriptionsrate gegenüber der Transkription vom gleichen Promotor bei ausreichend vorhandenem bioverfügbaren Stickstoff, Phosphor und/oder Schwefel verstanden. Markergene im Sinne der Erfindung sind Gene, die für Genprodukte kodieren, deren Aktivität zu einer physikalisch messbaren Änderung führt. Diese physikalisch messbare Änderung ist abhängig von der Änderung der Transkriptionsrate. Dies kann durch ein geeignetes Nachweissystem einfach und schnell detektiert werden. Bevorzugt werden solche Markergene verwendet, deren Genprodukte nachgewiesen werden können, ohne die Integrität bzw. Vitalität der Zellen zu beeinträchtigen.

Bei den Genprodukten handelt es sich bevorzugt um Proteine. Bevorzugt verwendet werden Markergene, deren Genprodukt ein Enzym ist, das in Anwesenheit eines Substrats eine Farbreaktion katalysiert. Weiter bevorzugt werden Markergene, die für eine Luciferase kodieren, die in Anwesenheit eines Substrats Licht aussendet. Besonders bevorzugt werden Markergene, deren Genprodukt ein Protein ist, das durch Anregung mit Licht einer bestimmten Wellenlänge fluoresziert.

Als Promotor wird in der Genetik eine DNA-Sequenz bezeichnet, die die Expression eines Gens reguliert. Promotoren im Sinne der Erfindung sind bevorzugt diejenigen Bereiche der genomischen DNA, die spezifisch für die Regulation der Expression eines Gens verantwortlich sind, indem sie auf spezifische intra- oder extrazelluläre Signale reagieren und abhängig von diesen Signalen die Expression des unter ihrer Kontrolle liegenden Gens aktivieren oder reprimieren. Im erfindungsgemäßen Verfahren sind diese Signale der Mangel an bioverfügbarem Stickstoff, Schwefel und/oder Phosphor. In Hefen befinden sich diese regulierenden DNA-Bereiche in der Regel auf der 5 '-Seite des Startcodons des betreffenden Gens und haben eine durchschnittliche Länge von 309 bp (Mewes H. W. et al., Overview of the yeast genome. Nature (1997) 387, 7-65). Solche regulierenden Bereiche können aber auch weiter als 1000 bp entfernt von der kodierenden Sequenz oder auf der 3'-Seite der kodierenden Sequenz des betreffenden Gens oder sogar innerhalb der transkribierten Sequenz des betreffenden Gens liegen. Setzt man derartige Promotoren an die 5'-Seite des Startcodons eines beliebigen Gens, bevorzugt eines Markergens, regulieren sie die Aktivität dieses Gens in Abhängigkeit von den oben genannten spezifischen Signalen.

Das unter die Kontrolle eines Promotors eines Gens, dessen Transkription sich bei Stickstoff-, Phosphor- oder Schwefel-Mangel stark erhöht oder stark erniedrigt, gestellte Markergen wird in eine Hefezelle eingebracht. Dabei kann es in der Hefezelle auf einem extrachromosomalen DNA-Molekül vorliegen. Bevorzugt verwendet wird dazu ein Hefe-Expressionsvektor, der bei der Teilung der Hefezelle stabil repliziert wird. Besonders bevorzugt ist ein sogenannter "high copy number" Vektor, der in der Hefezelle in einer großen Anzahl an Kopien vorliegt. Alternativ werden als extrachromosomale DNA-Moleküle auch künstliche Hefechromosomen (yeast artificial chromosomes) verwendet.

In einer anderen Ausführungsform wird das Markergen zusammen mit dem Promotor in die chromosomale DNA der Hefezelle integriert. Dadurch wird vorteilhaft sichergestellt, dass alle Nachkommen der Hefezelle ebenfalls das Markergen unter Kontrolle des spezifischen Promotors enthalten.

Um die physikalisch messbare Änderung, die durch die Aktivität der Markergene bewirkt wird, zu detektieren, sind die gentechnisch veränderten Hefezellen mit wenigstens einem Signaldetektor gekoppelt. Die Art des Signaldetektors bestimmt sich durch die Art der physikalisch messbaren Änderung. Als Signaldetektor wird bevorzugt ein Fotodetektor oder ein Spektrometer eingesetzt.

Der erfindungsgemäße Sensor ist äußerst robust: Stellt man in einer Zelle z. B. die Expression von zwei unterschiedlich fluoreszierenden Proteinen, deren Fluoreszenzen messtechnisch gut trennbar sind, zum einen unter die Kontrolle eines Promotors, welcher die Transkription bei einer Limitierung spezifisch stark erhöht, und zum anderen in der gleichen Zelle die Expression des zweiten Proteins unter die Kontrolle eines Promotors, der die Transkription stark erniedrigt, wird die Möglichkeit eines Messfehlers stark reduziert.

In dem erfindungsgemäßen Ganzzellsensor sind die Hefezellen in Xerogelen immobilisiert. Xerogele sind Gele, die ihre Flüssigkeit beispielsweise durch Verdampfen oder Absaugen verloren haben. Gele sind formbeständige, leicht deformierbare disperse Systeme aus mindestens zwei Komponenten, die zumeist aus einem festen Stoff mit langen oder stark verzweigten Teilchen (z.B. Kieselsäure, Gelatine, Kollagene, Polysaccharide, Pektine, spezielle Polymere, wie z.B. Polyacrylate, und andere, oft als Verdickungsmittel bezeichnete Geliermittel) und einer Flüssigkeit (meist Wasser) als Dispersionsmittel bestehen. Dabei bildet die feste Substanz im Dispersionsmittel ein räumliches Netzwerk. Bei der Entstehung von Xerogelen verändert sich die räumliche Anordnung des Netzes.

Die erfindungsgemäße Verwendung von anorganischen oder biologisch inerten organischen Xerogelen zur Einbettung der Hefezellen erlaubt vorteilhaft das Überleben der Zellen bei gleichzeitiger Stabilität der erzeugten Strukturen, denn sie sind toxikologisch und biologisch inert und werden im Allgemeinen nicht durch die Hefen abgebaut. Sie ermöglichen weiterhin vorteilhaft die Einlagerung von Nährstoffen und Feuchthaltemitteln, die das Überleben der Zellen sichern.

Vorteilhafte Ausgestaltungen der Erfindung sind in den Patentansprüchen 2 bis 22 angegeben.

Die Hefezellen sind erfindungsgemäß in einem porösen und optisch transparenten anorganischen oder biologisch inerten organischen Xerogel immobilisiert. Nach der Weiterbildung des Patentanspruchs 2 ist das Xerogel ein anorganisches Xerogel aus Siliziumdioxid, alkyliertem Siliziumdioxid, Titandioxid, Aluminiumoxid oder deren Gemischen, nach der Weiterbildung des Patentanspruchs 3 wird das anorganische Xerogel vorzugsweise durch einen Sol-Gel-Prozess hergestellt.

Dazu werden zunächst Silica- oder andere anorganische Nanosole entweder durch säure- oder alkalikatalysierte Hydrolyse der entsprechenden Silizium- oder Metallalkoxide in Wasser oder einem wasserlöslichem organischem Lösungsmittel (wie Ethanol) hergestellt. Bevorzugt wird die Hydrolyse in Wasser durchgeführt, um toxische Effekte des Lösungsmittels auf die einzubettenden Zellen zu verhindern. Bei der Herstellung von Nanosolen durch Alkoxidhydrolyse entstehen im Zuge der Reaktion Alkohole, die anschließend aus dem erhaltenen Nanosol durch Durchleitung eines inerten Gasstroms verdampft und durch Wasser ersetzt werden.

Durch die Verwendung von Gemischen verschiedener Alkoxide können die Matrixeigenschaften gezielt beeinflusst werden. Die Sol-Gel-Matrix erlaubt vorteilhaft die chemische Modifizierung durch Co-Hydrolyse und Co-Kondensation unter Verwendung verschiedener Metalloxide von Metallen wie Al, Ti, Zr zur Herstellung von gemischten Oxiden oder von Alkoxysilanen mit organischen Resten am Si-Atom zur Herstellung von organisch modifizierten Siliziumoxid-Gelen.

Die einzubettenden Zellen werden mit dem entstandenen Nanosol gemischt. Der Prozess der Gelbildung wird bevorzugt eingeleitet durch Erhöhung der Temperatur, Neutralisierung des pH-Werts, Aufkonzentrierung oder die Zugabe von Katalysatoren wie beispielsweise Fluoriden. Dabei sollte die Temperatur jedoch nicht auf Temperaturen >42°C erhöht werden, um die einzubettenden Zellen nicht zu schädigen. Bei der Überführung in ein Gel verringern die Nanosole ihr Oberflächen/Volumenverhältnis durch Aggregation und dreidimensionale Quervernetzungen. Während dieser Umwandlung des Nanosols in ein sogenanntes Lyogel werden die Zellen in dem entstehenden anorganischen Netzwerk immobilisiert. Die Immobilisierung überlebensfähiger Zellen wird vorteilhaft durch das Verhältnis Hefezellen:Oxid und durch die Zugabe von Poren-formenden Agentien gesteuert.

Der Anteil von Hefezellen an der Gesamtmenge des erzeugten Xerogels einschließlich der eingebetteten Zellen kann je nach Anwendung von 0,1 bis 50% Gewichtsprozent betragen. Bevorzugt verwendet wird ein Anteil von 2 bis 25% Gewichtsprozent.

Durch Trocknung wird dem Lyogel das noch enthaltene Lösungsmittel entzogen. Dadurch bildet sich aus dem Lyogel das Xerogel. Das entstehende Xerogel weist eine hohe Porosität auf, die einen raschen Stoffaustausch mit dem umgebenden Medium erlaubt. Der Trocknungsprozess hat eine starke Schrumpfung des Gels zur Folge, der zu Stress für die eingebetteten Zellen führt. Bevorzugt wird der Trocknungsschritt daher sehr schonend und langsam bei Temperaturen von weniger als 40°C durchgeführt.

Mit sinkendem Wassergehalt der Matrix verringern sich die physiologische Aktivität und die Überlebensrate der eingebetteten Zellen. Ein zu hoher Wassergehalt führt jedoch zu niedriger mechanischer Stabilität und verringert die Haltbarkeit der Struktur.

Die erfindungsgemäße Verwendung von Hefezellen ist daher besonders vorteilhaft, da Hefezellen eine hohe Resistenz gegen Trockenheit besitzen und auch bei sehr geringem Wassergehalt ihre Überlebensfähigkeit nicht einbüßen. Dadurch wird es möglich, sehr trockene Xerogele herzustellen.

Die Erfindung umfasst auch die Verwendung verschiedener Additive wie lösliche organische Salze, d. h. Metallsalze organischer Carbon- oder Sulfonsäuren bzw. offenkettige oder cyclische Ammoniumsalze und Quartärsalze von N-Heterocyclen sowie niedermolekulare Polyanionen oder Polykationen, oder wasserlösliche organische Verbindungen wie Polycarbonsäuren, Harnstoff-Derivate, Kohlenhydrate, Polyole, wie Glycerin, Polyethylenglycol und Polyvinylalkohol, oder Gelatine, die als Weichmacher, Feuchthaltemittel und Porenbildner wirken, die Zelllyse hemmen und die Überlebensfähigkeit der eingebetteten Zellen beträchtlich verlängern.

Das Xerogel ist mit den Hefezellen nach der Weiterbildung des Patentanspruchs 4 auf einem Substrat aufgebracht. In Verbindung mit dem Signaldetektor, vorzugsweise einem Fotodetektor, ist damit ein Funktionselement vorhanden, wobei das in Abhängigkeit vom bioverfügbaren Analyten erzeugte Fluoreszenzlicht über den Fotodetektor in ein elektrisches Signal umgewandelt wird. In Fortführung ist das Substrat vorteilhafterweise nach der Weiterbildung des Patentanspruchs 5 eine Lichtleitfaser, Glasbeads, ein planarer Glasträger oder andere Formkörper aus Glas wie Hohlkugeln, Stäbe, Röhren oder keramische Granulate.

Dabei werden die Hefezellen in einem porösen und optisch transparenten Xerogel, z. B. einem Siliziumdioxid-Xerogel, fixiert. Das mit den Mikroorganismen versetzte Siliziumdioxid-Xerogel wird als Schicht auf Glasbeads, einer Lichtleitfaser, planaren Glasträgern oder anderen Formkörpern wie Hohlkugeln, Stäben, Röhren oder keramischen Granulaten mittels eines bekannten Sol-Gel-Prozesses abgeschieden, indem das Nanosol-Zell-Gemisch auf das zu beschichtende Substrat aufgebracht oder das Substrat in das Nanosol-Zell-Gemisch eingetaucht wird und das Nanosol anschließend durch Trocknung und die dadurch resultierende Aufkonzentrierung des Nanosols in ein Xerogel überführt wird. Die dadurch vorhandene mechanische Stabilität dieser Strukturen erlaubt das Einbringen des Ganzzellsensors in ein Messsystem, das unmittelbar mit dem zu untersuchenden Reaktionsraum (Fermenter) im Sinn einer near-line-Diagnostik verbunden werden kann.

Die Hefezellen sind nach der Weiterbildung des Patentanspruchs 6 ein Bestandteil einer einen Hohlraum wenigstens teilweise umschließenden Hüllenstruktur. Das heißt, dass einzelne oder mehrere Hefezellen in diesem Hohlraum, der eine poröse Hülle hat, eingekapselt werden. Die Mikroporosität erlaubt vorteilhaft einen Stoffaustausch mit der Umgebung. In Fortführung besteht die Hüllenstruktur nach der Weiterbildung des Patentanspruchs 7 vorteilhafterweise aus einem Grundkörper mit einer inneren Schicht aus einem biologischen Hydrogel und einer äußeren Schicht aus dem porösen und optisch transparenten Xerogel, wobei die Schichten wenigstens bereichsweise aufgebracht sind.

Die Hefezellen sind dabei in der Hüllenstruktur eingebettet (Duplex-Einbettung). Die innere Hülle besteht aus einem biologischen Hydrogel, beispielsweise Alginat, und die äußere Hülle ist eine poröse Xerogel-Schicht, bevorzugt eine anorganische Xerogel-Schicht, besonders bevorzugt eine Siliziumdioxid-Xerogel-Schicht. Das biologische Hydrogel stabilisiert vorteilhaft die Hefezellen bei dem nachträglichen Prozess der Beschichtung mit dem Siliziumdioxid-Sol und erhöht somit die Überlebenswahrscheinlichkeit der Zellen. Diese Duplex-Einbettung kann vorteilhafterweise mittels einer sequentiellen Beschichtung unter Nutzung eines Nanoplotters erfolgen. Die mechanische Stabilität solcher Strukturen erlaubt das Einbringen des Ganzzellsensors in den zu untersuchenden Reaktionsraum (Fermenter) im Sinn einer near-line-Diagnostik.

Die Hefezellen befinden sich nach der Weiterbildung des Patentanspruchs 8 wenigstens auf einer Oberfläche in einer transparenten Messzelle. Diese besitzt darüber hinaus Einrichtungen zum Zuführen und Abführen des Mediums. Nach der Weiterbildung des Patentanspruchs 9 kann die Messzelle mit einer Heizeinrichtung gekoppelt sein.

Dabei befinden sich die Hefezellen in einer temperierbaren und lichtmikroskopisch beobachtbaren Messzelle eines Mikrofluidiksystems. Das Einbringen des Mediums in die Messzelle erfolgt mittels eines Mikrofluidiksystems (off-line-Diagnostik). Die Temperatureinstellung ist dabei unabhängig von der Temperatureinstellung im Fermenter.

Nach der Weiterbildung des Patentanspruchs 10 ist der Signaldetektor ein Fotodetektor. Der Fotodetektor ist ein Festkörperbildsensor mit Fotowiderständen, Fotodioden oder Fototransistoren, wobei dieser mit einem Datenverarbeitungssystem zusammengeschaltet ist. Ein Festkörperbildsensor ist eine flächenhafte und matrixförmige Anordnung optoelektronischer Halbleiterbauelemente als lichtelektrische Empfänger. Die Farbe und deren Intensität der Hefezellen sind in äquivalente elektrische Signale wandelbar, so dass eine Verarbeitung im Datenverarbeitungssystem stattfinden kann.

Im Strahlengang zwischen den Hefezellen und dem Fotodetektor befindet sich nach der Weiterbildung des Patentanspruchs 11 wenigstens eine Linse. Dadurch können die Lichtstrahlen der Hefezellen auf den Fotodetektor fokussiert werden, so dass eine sichere Auswertung auch lichtschwacher Änderungen gegeben ist.

Die Hefezellen sind nach der Weiterbildung des Patentanspruchs 12 mit einer optischen Strahlungsquelle so gekoppelt, dass die Strahlung auf die Hefezellen gelangt und die Hefezellen fluoreszieren. Die Strahlungsquelle liefert vorzugsweise elektromagnetische Strahlen als Licht im Sichtbaren und den angrenzenden Wellenlängenbereichen im Infraroten oder Ultravioletten. Vorzugsweise ist das eine elektromagnetische Strahlungsquelle, die Licht mit einer definierten Wellenlänge aussendet. Die Wellenlänge der Strahlungsquelle richtet sich nach dem Anregungsspektrum der fluoreszierenden Proteine.

Das Markergen ist nach der Weiterbildung des Patentanspruchs 13 unter die Kontrolle eines Promotors gestellt, der ausgewählt ist aus den Promotoren der Gene *YIR028W, YJR152W, YKR034W, YAR071W, YHR136C, YFL055W, YLL057C, NSR1, FET3, HIPI, YDR508C, RPS22B, YBR099C, IPTI, SSU1, SOL1* und *CTR1* von *Saccharomyces cerevisiae.*

Promotoren im Sinne der Erfindung sind auch DNA-Abschnitte, die zu den entsprechenden Hefe-Promotoren eine Homologie von mehr als 50%, bevorzugt mehr als 80%, aufweisen. Diese Abschnitte können beispielsweise aus homologen genomischen Bereichen von anderen Organismen, bevorzugt anderen Hefestämmen, stammen. Sie können jedoch auch synthetisch hergestellte DNA-Sequenzen sein, deren Sequenz eine Homologie von mehr als 50%, bevorzugt mehr als 80%, Übereinstimmung mit dem entsprechenden *Saccharomyces cerevisiae* Promotor aufweist. Promotoren können auch synthetische DNA-Sequenzen sein, die aus einem Teilbereich eines der oben genannten Hefe-Promotoren sowie einem bekannten Basalpromotor aus *Saccharomyces cerevisiae* zusammengesetzt sind. Der Basalpromotor stellt die für die Anbindung der Transkriptionsmaschinerie notwendigen DNA-Sequenzen zur Verfügung, während die Teilsequenzen aus den Hefepromotoren spezifisch auf regulierende Signale reagieren. Ein solcher Basalpromotor ist bevorzugt der Basalpromotor des *Cytochrom* c-Gens aus *Saccharomyces cerevisiae,* der 300 bp 5'seitig des Startcodons des *Cytochrom c-*Gens umfasst (Chen, J., et al. Binding of TFIID to the yeast CYC1 TATA boxes in yeast occurs independently of upstream activating sequences. PNAS (1994) 91:11909-11913).

Promotoren aus synthetischen DNA-Sequenzen können auch mehrfache Abschnitte einer identischen DNA-Sequenz enthalten. Diese Vervielfachung eines regulatorischen DNA-Abschnitts erlaubt vorteilhaft eine Erhöhung der Sensitivität des Promotors gegenüber den zu detektierenden Signalen.

Promotoren von Genen, deren Transkription als Antwort auf eine entsprechende Limitierung stark erhöht ist, sind vorteilhafterweise bei einer
- Stickstoff-Limitierung: *YIR028W, YJR152W* und *YKR034W*
- Phosphor-Limitierung: *YAR071W* und *YHR136C* und
- Schwefel-Limitierung: *YFL055W* und *YLL057C.*

Promotoren von Genen, deren Transkription als Antwort auf eine entsprechende Limitierung stark erniedrigt ist, sind vorteilhafterweise bei einer
- Stickstoff-Limitierung: *NSR1*, *FET3*, *HIP1* und *YDR508C*
- Phosphor-Limitierung: *RPS22B*, *YBR099C* und *IPT1*
- Schwefel-Limitierung: *SSU1*, *SOL1* und *CTR1*.

Bevorzugt wird als Promotor ein DNA-Abschnitt verwendet, der bis zu 1000 bp 5'-seitig des Startcodons des von ihm kontrollierten Gens umfasst, oder ein Teilabschnitt dieses DNA-Abschnitts, der in der Lage ist, auf die Limitierung von Stickstoff, Phosphor oder Schwefel hin das unter Kontrolle dieser Sequenz liegende Markergen zu aktivieren oder zu unterdrücken.

Besonders bevorzugt als Promotoren sind diejenigen genomischen Bereiche, die von den in Tabelle 1 aufgeführten Primerpaaren umschlossen werden. Die unterstrichenen Bereiche der Sequenzen sind dabei komplementär zu Bereichen der zu amplifizierenden DNA; die nicht unterstrichenen Bereiche der Primer enthalten Schnittstellen für Restriktionsenzyme zur anschließenden Subklonierung der erhaltenen PCR-Fragmente.

**Tab. 1**

| | | | | | |
|---|---|---|---|---|---|
| | | **forward-Primersequenz** | **Seq.- Nr.** | **reverse-Primersequenz** | **Seq.- Nr.** |
| **Stickstoff-limitierung** | *YIR028W* | | 1 | | 2 |
| | *NSR1* | | 3 | | 4 |
| **Phosphor- limitierung** | *YAR071 W* | | 5 | | 6 |
| | *RPS22B* | | 7 | | 8 |
| **Schwefel- limitierung** | *YFL055W* | | 9 | | 10 |
| | *SSU1* | | 11 | | 12 |

Nach der Weiterbildung des Patentanspruchs 14 kodiert das Markergen für ein Enzym, das durch eine einfache Farbreaktion nachweisbar ist, z. B. β-Galaktosidase, Alkalische Phosphatase, Meerrettich-Peroxidase. Die Erfindung umfasst auch Markergene, die für Enzyme kodieren, die zu einer Ansäuerung des Mediums führen. Der pH-Shift wird durch ein fluoreszierendes Protein, dessen Fluoreszenz vom pH-Wert der Umgebung abhängig ist, in ein Signal umgewandelt, das durch einen Photodetektor erfasst wird. Bevorzugt verwendet werden dazu pHluorine. Nach der Weiterbildung des Patentanspruchs 15 kodiert das Markergen für eine Luciferase. Luciferasen sind Proteine, die zur Biolumineszenz befähigt sind und in Anwesenheit von Luciferinen Licht abgeben, das dann vom Fotodetektor detektiert wird.

Weiterhin umfasst die Erfindung Markergene, die für Proteasen kodieren, die fluoreszierende Proteine abbauen. Dadurch wird auf das zu detektierende Signal hin die Abnahme der Fluoreszenz des Ganzzellsensors messbar. Bevorzugt verwendet werden Proteasen, die außer dem fluoreszierenden Protein keine weiteren Ziele in der Hefezelle angreifen, um die Vitalität der Hefezelle nicht zu beeinträchtigen. Besonders bevorzugt verwendet wird die TEV-Protease. Die entsprechenden fluoreszierenden Proteine müssen ggf. mittels rekombinanter DNA-Techniken so verändert werden, dass sie die Erkennungssequenz für die entsprechende Protease enthalten und so abbaubar sind.

Nach der Weiterbildung des Patentanspruchs 16 kodiert das Markergen für ein fluoreszierendes Protein, wobei die Expression des entsprechenden Markerproteins bei einer Limitierung von bioverfügbarem Stickstoff, Phosphor und/oder Schwefel im Medium variiert, was zu einer Zu- oder Abnahme der Fluoreszenz der jeweiligen Hefezelle führt. Bevorzugt verwendet werden die für die fluoreszierende Proteine GFP, YFP, CFP, BFP, RFP, DsRed, PhiYFP, JRed, emGFP ("Emerald Green"), Azami-Green, Zs-Green oder AmCyan 1 kodierenden Gene. Bevorzugt verwendet werden Proteine, die so verändert wurden, dass sie besonders stark fluoreszieren wie eGFP, eYFP, TagCFP, TagGFP, TagYFP, TagRFP und TagFP365. Weiterhin werden bevorzugt solche fluoreszierenden Proteine verwendet, deren Aminosäuresequenz dahingehend verändert wurde, dass sie möglichst schnell nach ihrer Bildung beginnen zu fluoreszieren. Bevorzugt verwendet werden dabei TurboGFP, TurboYFP, TurboRFP, TurboFP602, TurboFP635, und dsRed-Express.

Nach der Weiterbildung des Patentanspruchs 17 kodiert das Markergen für ein grün, (z. B. GFP), gelb (z. B. YFP), blau (z. B. BFP), cyan (z. B. CFP) oder rot (z. B. dsRed) fluoreszierendes Protein. Nach der Weiterbildung des Patentanspruchs 18 kodiert das Markergen für ein fluoreszierendes Protein mit eingeschränkter Halbwertszeit. Dadurch wird eine rasche Ansprechzeit bei einer Abnahme der Transkription gewährleistet.

Eine solche eingeschränkte Halbwertszeit kann zum Beispiel durch die Veränderung der N-terminalen Aminosäure oder das Einbringen einer Signalsequenz in die Aminosäuresequenz des vom Markergen kodierten Proteins erreicht werden, wodurch die Stabilität des Proteins gesenkt und seine Halbwertszeit verkürzt wird. Bevorzugt verwendet wird für die Destabilisierung des vom Markergen kodierten Proteins eine sogenannte PEST-Domäne, die zu einem raschen Abbau des Proteins durch das Ubiquitin-System der Zelle führt. Solche PEST-Domänen sind aus vielen Proteinen bekannt. Bevorzugt verwendet wird die PEST-Domäne des G1 Cyclins Cln2p aus *Saccharomyces cerevisiae.* Hierfür wird an das 3'-Ende der kodierenden Sequenz des Markergens die kodierende Sequenz (Seq.-Nr. 13) der 178 carboxyterminalen Aminosäuren von Cln2p (Seq.-Nr. 14) und ein Stoppcodon angefügt.

Nach der Weiterbildung des Patentanspruchs 19 werden als Hefezellen solche Zellen eingesetzt, die genetisch dergestalt verändert wurden, dass ihr Wachstum gezielt gesteuert werden kann. Dies erlaubt vorteilhaft, die für die Herstellung von Biosensoren benötigte Menge an Hefezellen unter sogenannten permissiven Bedingungen heranzuziehen und nach der Einbettung der Hefezellen die Hefezellen durch die Einstellung von restriktiven Bedingungen daran zu hindern, sich weiter zu teilen. Dadurch wird der durch das vegetative Wachstum der Zellen innerhalb der Matrix ausgeübte Druck vorteilhaft vermieden, der sowohl die Haltbarkeit der Biosensoren beeinträchtigt als auch Stress auf die immobilisierten Zellen ausübt und deren Vitalität negativ beeinflusst.

Bevorzugt eignen sich Hefezellen, bei denen die Aktivität eines Gens, welches auf den Zellzyklus einwirkt, gezielt gesteuert werden kann. Besonders bevorzugt werden Hefezellen, bei denen die Aktivität des *cdc28*-Gens gezielt gesteuert werden kann. Das *cdc28*-Gen wird von der Hefezelle benötigt, um sich teilen zu können. Ist das Gen nicht vorhanden, kann die Hefezelle zwar überleben, aber sich nicht weiter teilen.

Die Steuerung der Genaktivität erfolgt beispielsweise durch das sogenannte Tet on-System. Dabei wird eine Hefezelle, in der das endogene *CDC28*-Gen deletiert ist (eine sogenannte Δ*cdc28*-Zelle), mit einem DNA-Konstrukt transformiert, dass die kodierende Sequenz des *CDC28*-Gens unter der Kontrolle eines tet-responsiven Promotors enthält. Gleichzeitig enthält das Konstrukt die kodierende Sequenz des reversen Tetracyclin-kontrollierten Transaktivators (rtTA) unter der Kontrolle eines konstitutiven Promotors.
Solche genetisch veränderten Hefezellen exprimieren ständig den reversen Tetracyclinkontrollierten Transaktivator. Dieser kann nur in Gegenwart eines Tetracyclin-Antibiotikums wie beispielsweise Doxycyclin an den tet-responsiven Promotor binden und die Expression des unter der Kontrolle des tet-responsiven Promotors stehenden Gens unterdrücken. Um die Zellen heranzuziehen, setzt man dem Nährmedium ein Tetracyclin-Antibiotikum zu und stellt somit permissive Bedingungen her. Bei bzw. nach der Einbettung der Hefezellen in das Xerogel wäscht man das Tetracyclin-Antibiotikum aus und schafft dadurch restriktive Bedingungen für die Hefe. Der reverse Tetracyclin-kontrollierte Transaktivator kann nicht mehr die Expression des *CDC28*-Gens aktivieren. Die Hefezellen können sich also nicht mehr weiter teilen.

Nach der Weiterbildung des Anspruchs 20 werden als Hefezellen Zellzyklus-(*cdc*; cell division cycle)-Hefe-Mutanten eingesetzt, welche bei permissiver Temperatur normal wachsen und bei restriktiver Temperatur das Wachstum einstellen. So sind mehrere temperatursensitive (ts) Allele des *CDC28*-Gens aus *Saccharomyces cerevisiae* bekannt. Es wurden z.B. sechs verschiedene ts-Allele identifiziert, welche ein normales Wachstum der Hefen bei 23°C erlauben, jedoch das Wachstum bei 37°C verhindern (Lörincz and Reed, 1986). Weiterhin sind auch solche temperatursensitiven Mutationen bekannt, bei denen die permissive Temperatur höher ist als die restriktive Temperatur. Diese bezeichnet man als kältesensitive (*cold sensitive*, *cs*) Mutationen.

Vorteilhaft kann durch die Nutzung solcher Mutanten bei permissiver Temperatur zunächst die benötigte Biomasse erzeugt werden, während die Hefezellen das Wachstum bei restriktiver Temperatur einstellen. Werden solche Mutanten für Ganzzellsensoren eingesetzt, so können bei thermosensitiven Mutanten die Zellen vorteilhaft bis zum Erreichen der gewünschten Biomasse bei ca. 25°C angezogen und dann eingebettet werden. Bei restriktiver Temperatur von z. B. 37°C - einer Temperatur wie sie für Fermentation von *Escherichia coli* ideal ist - erfolgt kein Wachstum der Hefen mehr, obwohl die Zellen physiologisch aktiv sind. (Lörincz, A. and Reed, S.I. Sequence analysis of temperature-sensitive mutations in the Saccharomyces cerevisiae gene CDC28. Mol. Cell. Biol. (1986) 6:4099-4103) Bevorzugt verwendet werden Hefen, die die temperatursensitiven Allele *cdc28-4, cdc28-6*, *cdc28-9, cdc28-13*, *cdc28-16*, *cdc28-17*, *cdc28-18* und *cdc28-19* tragen.
Für Anwendungen, bei denen die Hefen in niedrigeren Temperaturen wie beispielsweise Raumtemperatur eingesetzt werden sollen, werden kältesensitive Mutanten eingesetzt, die bei hohen Temperaturen herangezogen werden und nach der Einbettung bei niedriger Temperatur gehalten werden und so keine Teilungsaktivität mehr aufweisen.

Nach der Weiterbildung des Patentanspruchs 21 wird eine Kombination aus einem grün (z. B. GFP), einem gelb (z. B. YFP), einem blau (z. B. BFP), einem cyan (z. B. CFP) und/oder einem rot (z. B. dsRed) fluoreszierenden Markerprotein verwendet. Bevorzugt verwendet man Kombinationen von Proteinen, deren Anregungs- und Emissionsspektren messtechnisch klar voneinander trennbar sind. Besonders bevorzugt sind die Kombinationen GFP und YFP sowie GFP und DsRed. Die Expression des entsprechenden Markergens variiert bei einer Limitierung von bioverfügbarem Stickstoff, Phosphor und/oder Schwefel im Medium, das zu einer Zu- oder Abnahme der Fluoreszenzintensitäten in der jeweiligen Hefezelle führt. Damit sind Limitierungen verschiedener Komponenten gleichzeitig detektierbar. Dadurch sind multifunktionelle Ganzzell-Sensoren vorhanden.

Die Enden zweier Lichtleitfasern sind nach der Weiterbildung des Patentanspruchs 22 gleichzeitig das Substrat für die Hefezellen oder ein Substrat mit den Hefezellen ist an die Lichtleitfasern gekoppelt. Weiterhin ist an das andere Ende einer ersten Lichtleitfaser eine Strahlungsquelle und an das andere Ende der zweiten Lichtleitfaser der Fotodetektor gekoppelt, so dass mittels der Lichtstrahlen in der ersten Lichtleitfaser die Hefezellen zum Fluoreszieren angeregt und das durch den bioverfügbaren Analyten hervorgerufene Fluoreszenzlicht über die zweite Lichtleitfaser an den Fotodetektor gelangt, wobei keine Strahlung von der Strahlungsquelle zum Substrat gelangt.

Nach der Weiterbildung des Patentanspruchs 23 ist ein Ende einer Lichtleitfaser das Substrat für die Hefezellen oder das Ende der Lichtleitfaser ist an ein Substrat mit den Hefezellen gekoppelt. Weiterhin ist das andere Ende der Lichtleitfaser über einen Strahlumschalter entweder mit einer Strahlungsquelle oder dem Fotodetektor gekoppelt, so dass entweder die Anregungstrahlung der Strahlungsquelle über den Strahlumschalter und die Lichtleitfaser zum Substrat oder das Fluoreszenzlicht über die Lichtleitfaser und den Strahlumschalter auf den Fotodetektor gelangen.

Gemäß Patentanspruch 24 kann der Einsatz der erfindungsgemäßen Ganzzellbiosensoren für folgende Zwecke erfolgen:
Kontrolle und/oder Steuerung der Verfügbarkeit von bioverfügbarem Stickstoff, bioverfügbarem Phosphor und/oder bioverfügbarem Schwefel in Bioreaktoren mit Anwendungsmöglichkeiten im gesamten Bereich der "Weißen Biotechnologie";
Überwachung und/oder Steuerung von Systemen zur Reinigung von Trinkwasser, technischem Brauchwasser und/oder Abwässern von Stickstoff-, Phosphor- und Schwefelbelastung.

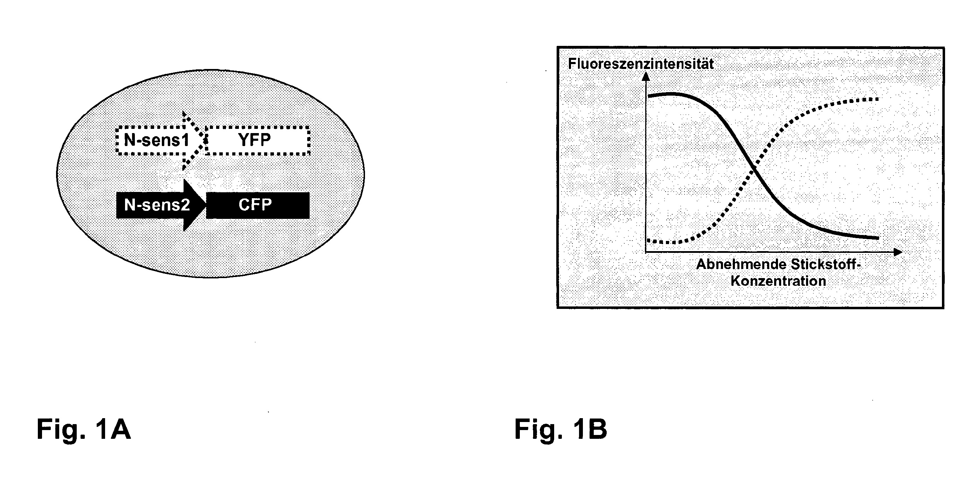

Anhand folgender Figuren und Ausführungsbeispiele wird die Erfindung näher erläutert. Dabei zeigt
**Fig. 1****:** Schematische Darstellung eines Ganzzellsensors

Figur 1 A zeigt schematisch eine Hefezelle, in der der für YFP kodierende Leserahmen (YFP) unter der Kontrolle eines Promotors (N-sens1) steht, welcher bei Stickstoffmangel zu einer erhöhten Transkription führt. Analog wird der Leserahmen von CFP unter die Kontrolle eines Promotors (N-sens2), der bei Stickstoffmangel zu einer verringerten Transkription führt, gestellt. Figur 1 B zeigt in einem Diagramm, wie anhand der jeweiligen Fluoreszenzen bzw. deren Verhältnis zueinander die aktuelle Verfügbarkeit von Stickstoff für die Zelle gemessen werden kann.

### Ausführungsbeispiel 1

Die Gene *YAR071W* und *RPS22B* werden bei einer Phosphorlimitierung jeweils spezifisch sehr viel stärker bzw. schwächer transkribiert. Die Klonierung von Konstrukten zur Detektion von Phosphorlimitierung ist im Folgenden beschrieben. Analog kann mit spezifischen Primern (siehe Tabelle 1) eine Klonierung für Konstrukte zur Detektion von Schwefel-limitierung erfolgen.

Die 1000 Basenpaare umfassende, stromaufwärtsliegende Region des heraufregulierten Gens *YAR071 W* wird mittels spezifischen Primern (siehe Tabelle 1) in einer PCR aus genomischer DNA von *Saccharomyces cerevisiae* amplifiziert. Durch die Primer wird die Sequenz um eine 5'-seitige Erkennungssequenz für *Sac*I und 3'-seitig um eine Erkennungssequenz für *Spe*I erweitert. Mittels dieser Erkennungssequenzen erfolgt ein gerichteter Einbau in den high copy-number Vektor p426, folgend p426YAR071W genannt. Für das herunterregulierte Gen *RPS22B* wird ebenfalls mit den in Tabelle 1 aufgeführten spezifischen Primern in einer PCR die regulatorische Region des Gens amplifiziert. Die amplifizierte Region umfasst die unmittelbar vor dem Leserahmen des Gens liegenden 1000 Basenpaare. Durch die verwendeten Primer werden an das *RPS22B*-Promotorfragment 5'-seitig und 3'-seitig Erkennungssequenzen für die Restriktionsendonukleasen *Sma*I und *Bam*HI angefügt. Der high copy-number Vektor p424GPD wird mit *Ecl*I und *Bam*HI geschnitten, wodurch der enthaltene *GPD*-Promotor entfernt wird. *Ecl*I und *Sma*I erzeugen stumpfendige Fragmente, die miteinander ligiert werden können: So erfolgt ein Einbau des *RPS22B-*Promotorfragmentes in das Plasmid, folgend p424RPS22B genannt.

Im zweiten Schritt wird der Leserahmen des Markergens unter die Kontrolle des *YAR071W-*bzw. *RPS22B*-Promotors in den Plasmiden p426YAR071W und p242RPS22B gebracht. Dazu wird die Sequenz des Markergens mit Primern amplifiziert, welche das Fragment 5'-seitig um eine *Spe*I- oder *Eco*RI- und 3'-seitig um eine *Sal*I-Schnittstelle erweitern. Unter die Kontrolle des *YAR071 W* -Promotors wird die kodierende Sequenz für YFP gestellt, unter die Kontrolle des *RPS22B*-Promotors wird die kodierende Sequenz für CFP gestellt. Danach erfolgt die Klonierung der entsprechenden Fragmente mit den genannten Restriktionsschnittstellen in die Vektoren p426YAR071W bzw. p424RPS22B. Die korrekte Sequenz der klonierten Fragmente wird mittels DNA-Sequenzanalyse überprüft und bestätigt.

Der Vektor p426 enthält das *URA3*-Gen und p424 das *TRP1*-Gen aus *Saccharomyces cerevisiae* zur Selektion in entsprechenden auxotrophen Stämmen. Die entstandenen Konstrukte (p426YAR071W bzw. p424RPS22B) werden in den Hefestamm W303 (*Mat a, ade2-1, his3-1, his3-15, leu-2-3, leu2-112, trp1-1, ura3-1)* transformiert und Transformanden selektiert. Bei Phosphorlimitierung wird in solchen Hefezellen die Transkription und Expression des YFP stark erhöht, die des CFP entsprechend erniedrigt.

Zur Fixierung der Hefezellen in einem porösen und optisch transparenten Siliziumdioxid-Xerogel wird ein Gemisch aus Hefezellen und einem Siliziumdioxid-Xerogel auf einer Lichtleitfaser mittels eines bekannten Sol-Gel-Prozesses abgeschieden.

Eine derartige Beschichtungslösung wird in folgender Weise hergestellt:
(1) Herstellung eines wässrigen SiO₂-Nanosols:
   100 ml Tetraethoxysilan, 400 ml Ethanol und 200 ml 0.01 M Salzsäure werden 20 h bei Raumtemperatur gerührt. Zu dieser Lösung werden 500 ml Wasser zugesetzt und ein starker Luftstrom solange durchgeleitet, bis ein Endvolumen von 700 ml erreicht ist. Das SiO₂-Sol (pH 3-4) enthält ca. 4.2 % SiO₂ mit einem mittleren Teilchendurchmesser von ca. 6 nm.
(2) Herstellung der Beschichtungslösung:
   In 100 ml des wässrigen SiO₂-Nanosols werden 2 g Hefezellen (Nassgewicht) unter Rühren bzw. Ultraschall 30 min. dispergiert. Als Dispergierhilfen können Netzmittel (z.B. 1 ml 5 % wässrige Tween 80-Lösung) zugesetzt werden. Mit dieser Zell-Suspension werden die obengenannten Glasträger durch einen dip-coating-Prozess (30cm/min) beschichtet und an der Luft getrocknet.

Die mechanische Stabilität dieser Strukturen erlaubt vorteilhaft die Kopplung des Ganzzellsensors mit dem zu untersuchenden Reaktionsraum (Fermenter) in einer Messzelle im Sinn einer near-line-Diagnostik. In speziellen Ausführungen wie einer Lichtleitfaser kann auch der Ganzzellsensor unmittelbar im Reaktionsraum (Fermenter) angebracht werden.

Die Endbereiche zweier Lichtleitfasern sind benachbart zueinander angeordnet. Die Enden der Lichtleitfasern sind gleichzeitig das Substrat für die Hefezellen. In einer Ausführungsform kann auch ein separates Substrat an die Lichtleitfasern angekoppelt sein. An das andere Ende einer ersten Lichtleitfaser ist die Strahlungsquelle und an das andere Ende der zweiten Lichtleitfaser ist der Fotodetektor gekoppelt. Mittels der Lichtstrahlen in der ersten Lichtleitfaser werden die Hefezellen zum Fluoreszieren angeregt. Das dadurch hervorgerufene Fluoreszenzlicht gelangt über die zweite Lichtleitfaser an den Fotodetektor, wobei keine Strahlung von der Strahlungsquelle zum Substrat gelangt. Dazu ist das Datenverarbeitungssystem mit einem Schalter für die Strahlungsquelle oder einer Blende für den Strahlengang nach der Strahlungsquelle zusammengeschaltet, so dass die Strahlung der Strahlungsquelle pulsiert zum Substrat gelangt. Der Fotodetektor ist mit dem Datenverarbeitungssystem zusammengeschaltet, so dass man über das über den Fotodetektor detektierte Fluoreszenzlicht im Verbund mit dem nachgeschalteten Datenverarbeitungssystem auf den Gehalt an bioverfügbaren Analyten rückschließen werden kann.

In einer Ausführungsform kann das Ende einer Lichtleitfaser das Substrat oder das Ende der Lichtleitfaser an das Substrat gekoppelt sein. Das andere Ende der Lichtleitfaser ist über einen Strahlumschalter entweder mit der Strahlungsquelle oder dem Fotodetektor gekoppelt, so dass entweder die Anregungstrahlung über den Strahlumschalter und die Lichtleitfaser zum Substrat oder das Fluoreszenzlicht über die Lichtleitfaser und den Strahlumschalter auf den Fotodetektor gelangen. Ein Strahlumschalter ist beispielsweise ein Klappspiegel. Der Fotodetektor und der Antrieb für den Klappspiegel sind mit dem Datenverarbeitungssystem zusammengeschaltet.

Die Strahlungsquelle ist dazu vorteilhafterweise eine elektromagnetische Strahlungsquelle für elektromagnetische Strahlen als Licht im Sichtbaren und den angrenzenden Wellenlängenbereichen im Infraroten oder Ultravioletten.

Das mit der/den Lichtleitfaser/n gekoppelte Substrat kann auch eine beschichtete transparente Röhrenstruktur sein, an die die Lichtleitfaser gekoppelt ist.

### Ausführungsbeispiel 2

Das Gen *YIR028W* wird bei Stickstofflimitierung spezifisch sehr viel stärker, das Gen *NSR1* bei Stickstofflimitierung spezifisch sehr viel schwächer transkribiert. Ausgehend von genomischer DNA von *Saccharomyces cerevisiae* werden mit spezifischen Primern jeweils die 5'-seitigen regulatorischen Bereiche der Gene einschließlich deren Promotoren PCR-amplifiziert (siehe Tabelle 1). Durch die Primer werden Schnittstellen an die Fragmente fusioniert, welche für die Klonierung in den Vektor pUC18 genutzt werden. Die korrekte Sequenz der klonierten Fragmente wird mittels DNA-Sequenzanalyse verifiziert.

Die Leserahmen des gelb fluoreszierenden Proteins (YFP) und des cyan fluoreszierenden Proteins (CFP) werden mit DNA der Plasmide pFA6a-EYFP bzw. pFA6a-ECFP (Driesche *et al.,* 2005) als Template PCR-amplifiziert. Mittels Overlap-Extension PCR (Pogulis *et al.*, 1996) wird der Leserahmen des YFP unter die Kontrolle des *YIR028W*-Promotors, der Leserahmen des CFP unter die Kontrolle des NSRI-Promotors gestellt. Die resultierenden Fragmente werden in die single-copy Vektoren pRS415 bzw. pRS416 (Sikorski and Hieter, 1989) kloniert. Der Vektor pRS415 trägt das *LEU2* und pRS416 das *URA3* Gen aus *S. cerevisiae* zur Selektion in entsprechenden auxotrophen Stämmen. Die entstandenen Konstrukte (pRS415*-YIR028W-YFP* und pRS416-*NSR1-CFP*) werden in den Hefestamm W303 (*Mat a, ade2-1, his3-1, his3-15, leu2-3, leu2-112, trp1-1, ura3-1*) transformiert und Transformanden selektiert. Bei Stickstofflimitierung wird in solchen Hefezellen die Transkription und Expression des YFP stark erhöht, die des CFP entsprechend erniedrigt.

Anschließend werden die Hefezellen in eine Hüllenstruktur eingebettet (Duplex-Einbettung), wobei die innere Hülle aus Alginat besteht und die äußere Hülle eine poröse Siliziumdioxid-Xerogel-Schicht ist. Die Duplex-Einbettung erfolgt mittels einer sequentiellen Beschichtung unter Nutzung eines Nanoplotters. Hierbei werden die Hefezellen vor dem Plotten in eine wässrige Alginat-Lösung (Alginat-Konzentration 2 Gewichtsprozent) eingebettet (der Hefezellenanteil variiert typischerweise zwischen 5 bis 25 Volumenprozent).

Durch den Nanoplotter werden definierte Volumina der in Alginat eingebetteten Hefezellen punktweise auf einen planaren Glasträger aufgebracht. Durch die Verwendung eines Nanoplotters kann über die Wahl der aufgetragenen Volumina sowie einer definierten Konzentration an Hefezellen gezielt die Mengen der auf den Glasträger aufgebrachten Hefezellen gewählt werden. Anschließend bringt der Nanoplotter auf die in Alginat eingebetteten Hefezellen eine Nanosolschicht auf, die anschließend durch Trocknung in ein Xerogel überführt wird.

Die mechanische Stabilität solcher Strukturen erlaubt vorteilhaft das Einbringen des Ganzellsensors in eine Messzelle, die unmittelbar mit dem zu untersuchenden Reaktionsraum (Fermenter) im Sinn einer near-line-Diagnostik gekoppelt ist.

### Ausführungsbeispiel 3

Die Hefezellen werden wie in Ausführungsbeispiel 1 oder 2 angegeben hergestellt. Anschließend werden die Hefezellen auf einem planaren Glasträger in eine temperierbare und lichtmikroskopisch beobachtbare Messzelle eines Mikrofluidiksystems aufgebracht. So wird ein gesteuertes Einbringen des zu analysierenden Mediums in die Messzelle über das Mikrofluidiksystem (off-line Diagnostik) ermöglicht. Vorteilhaft ist die Temperatureinstellung im Fermenter und in der Messzelle voneinander unabhängig.

## Patentansprüche

1. Ganzzellsensor zur Erfassung von bioverfügbarem Stickstoff, Phosphor und Schwefel jeweils einzeln oder in wenigstens einer Kombination in einem Medium,
bestehend aus gentechnisch veränderten Hefezellen, die in einer Xerogel-Matrix immobilisiert sind, wobei das Xerogel ein anorganisches Xerogel ist, das aus Siliziumdioxid, alkyliertem Siliziumdioxid, Titandioxid, Aluminiumoxid oder deren Gemischen besteht, und mit den Hefezellen auf einem Substrat aufgebracht ist,
wobei die Hefezellen mindestens ein Markergen unter der Kontrolle eines Promotors eines Gens enthalten, dessen Transkriptionsrate sich bei Stickstoff-, Phosphor- oder Schwefel-Mangel mindestens zweifach erhöht oder erniedrigt,
wobei die Hefezellen wenigstens mit einem Signaldetektor gekoppelt sind,
und wobei die Hefezellen ein Bestandteil einer einen Hohlraum wenigstens teilweise umschließenden Hüllenstruktur sind, die aus einem Grundkörper mit einer inneren Schicht aus einem biologischen Hydrogel und einer äußeren Schicht aus dem porösen und optisch transparenten Xerogel besteht, wobei die Schichten wenigstens bereichsweise aufgebracht sind.

2. Ganzzellsensor nach Anspruch 1, **dadurch gekennzeichnet, dass** das Xerogel durch Trocknung eines Lyogels bei einer Temperatur von weniger als 40 °C erhalten wurde.

3. Ganzzellsensor nach Anspruch 1 oder 2, **dadurch gekennzeichnet, dass** sich die Hefezellen wenigstens auf einer Oberfläche in einer transparenten Messzelle befinden und dass die Messzelle Einrichtungen zum Zuführen und Abführen des Mediums besitzt.

4. Ganzzellsensor nach einem der Ansprüche 1 bis 3, **dadurch gekennzeichnet, dass** der Signaldetektor ein Fotodetektor in Form eines Festkörperbildsensors mit Fotowiderständen, Fotodioden oder Fototransistoren ist und dass der Festkörperbildsensor mit einem Datenverarbeitungssystem zusammengeschaltet ist.

5. Ganzzellsensor nach einem der Ansprüche 1 bis 4, **dadurch gekennzeichnet, dass** das Markergen unter die Kontrolle eines Promotors gestellt ist, der ausgewählt ist aus den Promotoren der Gene *YIR028W, YJR152W, YKR034W, YAR071W, YHR136C, YFL055W, YLL057C, NSR1, FET3, HIP1, YDR508C, RPS22B, YBR099C, IPT1, SSU1, SOL1* und *CTR1* von *Saccharomyces cerevisiae*.

6. Ganzzellsensor nach einem der Ansprüche 1 bis 5, **dadurch gekennzeichnet, dass** das Markergen für ein Enzym kodiert, das durch eine einfache Farbreaktion nachweisbar ist.

7. Ganzzellsensor nach einem der Ansprüche 1 bis 6, **dadurch gekennzeichnet, dass** das Markergen für eine Luciferase kodiert.

8. Ganzzellsensor nach einem der Ansprüche 1 bis 7, **dadurch gekennzeichnet, dass** das Markergen für ein fluoreszierendes Protein kodiert, wobei die Expression des durch das Markergen kodierten Proteins bei einer Limitierung von bioverfügbarem Stickstoff, Phosphor und/oder Schwefel im Medium variiert, was zu einer Zu- oder Abnahme der Fluoreszenz der jeweiligen Hefezelle führt.

9. Ganzzellsensor nach Anspruch 8, **dadurch gekennzeichnet, dass** das Markergen für ein fluoreszierendes Protein mit eingeschränkter Halbwertszeit kodiert.

10. Ganzzellsensor nach einem der Ansprüche 1 bis 9, **dadurch gekennzeichnet, dass** als Hefezellen Zellzyklus-(*cdc*; cell division cycle)-Mutanten eingesetzt werden, welche bei permissiven Bedingungen normal wachsen und bei restriktiven Bedingungen das Wachstum einstellen.

11. Ganzzellsensor nach Anspruch 10, **dadurch gekennzeichnet, dass** als Hefezellen temperatursensitive Zellzyklus-(*cdc*; cell division cycle)-Mutanten eingesetzt werden, welche bei permissiver Temperatur normal wachsen und bei restriktiver Temperatur das Wachstum einstellen.

12. Verwendung eines Ganzzellsensors nach einem der Ansprüche 1 bis 11 zur Kontrolle und/oder Steuerung der Verfügbarkeit von bioverfügbarem Stickstoff, bioverfügbarem Phosphor und/oder bioverfügbarem Schwefel in Bioreaktoren oder zur Überwachung und/oder Steuerung von Systemen zur Reinigung von Trinkwasser, technischem Brauchwasser oder Abwässern von Stickstoff-, Phosphor- und Schwefelbelastung.

## Claims

1. A whole-cell sensor for detecting in a medium bio-available nitrogen, phosphorus, and sulfur, each individually or in at least one combination,
the whole-cell sensor consisting of gene-technologically modified yeast cells, which are immobilized in a xerogel matrix, wherein the xerogel is an inorganic xerogel, which consists of silicon dioxide, alkylated silicon dioxide, titanium dioxide, aluminum oxide, or mixtures thereof and which is applied onto a substrate together with the yeast cells,
wherein the yeast cells contain at least one marker gene under the control of a promoter of a gene whose transcription rate increases or decreases at least twofold in case of nitrogen deficiency, phosphorus deficiency or sulfur deficiency,
wherein the yeast cells are coupled at least to one signal detector,
and wherein the yeast cells are a component of an envelope structure that encloses at least partially a cavity, and that is comprised of a base body with an inner layer of a biological hydrogel and an outer layer of a porous and optically transparent xerogel, wherein the layers are applied at least section-wise.

2. The whole-cell sensor according to claim 1, **characterized in that** the xerogel has been obtained by drying of a lyogel at a temperature of less than 40 °C.

3. The whole-cell sensor according to claim 1 or 2, **characterized in that** the yeast cells are located at least on one surface in a transparent measuring cell and wherein the measuring cell has devices for supplying and removing the medium.

4. The whole-cell sensor according to one of the claims 1 to 3, **characterized in that** the signal detector is a photodetector in the form of a solid state image sensor with photoresistors, photodiodes or phototransistors and wherein the solid state image sensor is connected to a data processing system.

5. The whole-cell sensor according to one of the claims 1 to 4, **characterized in that** the marker gene is subjected to the control of a promoter that is selected from the promoters of the genes YIR028W, YJR152W, YKR034W, YAR071W, YHR136C, YFL055W, YLL057C, NSR1, FET3, HIP1, YDR508C, RPS22B, YBR099C, IPT1, SSU1, SOL1 and CTR1 of *Saccharomyces cerevisiae.*

6. The whole-cell sensor according to one of the claims 1 to 5, **characterized in that** the marker gene codes for an enzyme that is detectable by a simple color reaction.

7. The whole-cell sensor according to one of the claims 1 to 6, **characterized in that** the marker gene codes for a luciferase.

8. The whole-cell sensor according to one of the claims 1 to 7, **characterized in that** the marker gene codes for a fluorescent protein, wherein the expression of the protein that is coded by the marker gene varies in case of limitation of bio-available nitrogen, phosphorus and/or sulfur in the medium, leading to an increase or decrease of the fluorescence of the respective yeast cell.

9. The whole-cell sensor according to claim 8, **characterized in that** the marker gene codes for a fluorescent protein with limited half-life.

10. The whole-cell sensor according to one of the claims 1 to 9, **characterized in that** the yeast cells are cell division cycle (cdc) mutants that under permissive conditions grow normally and stop growth under restrictive conditions.

11. The whole-cell sensor according to claim 10, **characterized in that** the yeast cells are temperature-sensitive cell division cycle (cdc) mutants that under permissive temperature grow normally and stop growth under restrictive temperature.

12. Use of the whole-cell sensor according to one of the claims 1 to 11 to control and/or govern the availability of bio-available nitrogen, bio-available phosphorus and/or bio-available sulfur in bioreactors or to monitor and/or control systems for purifying drinking water, technical process water or waste water with regard to nitrogen, phosphorus, and sulfur loading.

## Revendications

1. Capteur entièrement cellulaire destiné à détecter de l'azote, du phosphore et du soufre biodisponibles respectivement individuellement ou au moins en combinaison dans un milieu, composé de cellules de levure génétiquement modifiées qui sont immobilisées dans une matrice de xérogel, le xérogel étant un xérogel inorganique qui se compose de dioxyde de silicium, de dioxyde de silicium alkylé, de dioxyde de titane, d'oxyde d'aluminium ou de leurs mélanges, et qui est appliqué sur un substrat avec les cellules de levure,
dans lequel les cellules de levure contiennent au moins un gène marqueur sous le contrôle d'un promoteur d'un gène dont le taux de transcription augmente ou diminue au moins du double en cas de carence en azote, en phosphore ou en soufre,
dans lequel les cellules de levure sont couplées avec au moins un détecteur de signal,
et dans lequel les cellules de levure sont un composant d'une structure d'enveloppe entourant au moins partiellement un espace creux, ladite structure se composant d'un corps de base présentant une couche interne d'un hydrogel biologique et une couche externe du xérogel poreux et optiquement transparent, les couches étant appliquées au moins par zone.

2. Capteur entièrement cellulaire selon la revendication 1, **caractérisé en ce que** le xérogel a été obtenu par séchage d'un lyogel à une température au moins égale à 40 °C.

3. Capteur entièrement cellulaire selon la revendication 1 ou 2, **caractérisé en ce que** les cellules de levure se trouvent au moins sur une surface d'une cellule de mesure transparente et **en ce que** la cellule de mesure possède des dispositifs permettant d'amener et d'évacuer le milieu.

4. Capteur entièrement cellulaire selon l'une des revendications 1 à 3, **caractérisé en ce que** le détecteur de signal est un photodétecteur sous la forme d'un capteur d'image à solide avec photorésistances, photodiodes ou phototransistors, et **en ce que** le capteur d'image à solide est interconnecté avec un système de traitement des données.

5. Capteur entièrement cellulaire selon l'une des revendications 1 à 4, **caractérisé en ce que** le gène marqueur est placé sous le contrôle d'un promoteur qui est sélectionné parmi les promoteurs des gènes *YIR028W*, *YJR152W*, *YKR034W, YAR071W*, *YHR136C*, *YFL055W*, *YLL057C, NSR1*, *FET3*, *HIP1*, *YDR508C*, *RPS22B*, *YBR099C*, *IPT1*, *SSU1*, *SOL1* et *CTR1* de *Saccharomyces cerevisiae.*

6. Capteur entièrement cellulaire selon l'une des revendications 1 à 5, **caractérisé en ce que** le gène marqueur code pour une enzyme qui peut être détectée par une réaction de coloration simple.

7. Capteur entièrement cellulaire selon l'une des revendications 1 à 6, **caractérisé en ce que** le gène marqueur code pour une luciférase.

8. Capteur entièrement cellulaire selon l'une des revendications 1 à 7, **caractérisé en ce que** le gène marqueur code pour une protéine fluorescente, suite à quoi l'expression de la protéine codée par le gène marqueur varie en cas de limitation de l'azote, du phosphore et/ou du soufre biodisponible(s) dans le milieu, ce qui génère une augmentation ou une réduction de la fluorescence de la cellule de levure respective.

9. Capteur entièrement cellulaire selon la revendication 8, **caractérisé en ce que** le gène marqueur code pour une protéine fluorescente présentant une demi-vie limitée.

10. Capteur entièrement cellulaire selon l'une des revendications 1 à 9, **caractérisé en ce que** l'on utilise comme cellules de levure des mutants cdc (cell division cycle) du cycle cellulaire qui dans des conditions permissives croissent normalement et dans des conditions restrictives arrêtent la croissance.

11. Capteur entièrement cellulaire selon la revendication 10, **caractérisé en ce que** l'on utilise en tant que cellules de levure des mutants cdc thermosensibles qui dans des conditions permissives croissent normalement et dans des conditions restrictives arrêtent la croissance.

12. Utilisation d'un capteur entièrement cellulaire selon l'une des revendications 1 à 11 pour contrôler et/ou commander la disponibilité de l'azote biodisponible, du phosphore biodisponible et/ou du soufre biodisponible dans des réacteurs biologiques ou pour surveiller et/ou commander des systèmes permettant de purifier la charge en azote, en phosphore et en soufre dans de l'eau potable, de l'eau industrielle ou des eaux usées.


### SEQUENCE LISTING

<110> Technische Universität Dresden
<120> Ganzzellsensor
<130> 17P0094DE
<160> 14
<170> PatentIn version 3.5
<210> 1
   <211> 42
   <212> DNA
   <213> Artificial Sequence
<220>
   <223> Primer
<400> 1
   tattatgagc tcgagatacg ttctccagcg tatgtatttc at 42
<210> 2
   <211> 49
   <212> DNA
   <213> Artificial Sequence
<220>
   <223> Primer
<400> 2
   tattatacta gttctcgtct ttgttgatgt tttatatcac aagatgtag 49
<210> 3
   <211> 42
   <212> DNA
   <213> Artificial Sequence
<220>
   <223> Primer
<400> 3
   tattatcccg gggagattcc aaactggttc attgaaatag gc 42
<210> 4
   <211> 41
   <212> DNA
   <213> Artificial Sequence
<220>
   <223> Primer
<400> 4
   tattatggat cccttatttt atcctgcctg ggttgagtga t 41
<210> 5
   <211> 36
   <212> DNA
   <213> Artificial Sequence
<220>
   <223> Primer
<400> 5
   tattatgagc tcggtgctgt gaccgtttcc aatacg 36
<210> 6
   <211> 44
   <212> DNA
   <213> Artificial Sequence
<220>
   <223> Primer
<400> 6
   tattatacta gttggtattt ctgatgatgt tcttgctctc tttg 44
<210> 7
   <211> 43
   <212> DNA
   <213> Artificial Sequence
<220>
   <223> Primer
<400> 7
   tattatcccg ggactgcaac tattcttaca atctttcatt tac 43
<210> 8
   <211> 45
   <212> DNA
   <213> Artificial Sequence
<220>
   <223> Primer
<400> 8
   tattatggat cctttttacc taattactat gttttgaaac gttag 45
<210> 9
   <211> 36
   <212> DNA
   <213> Artificial Sequence
<220>
   <223> Primer
<400> 9
   tattatgagc tctgttcacg ccctctacga accatg 36
<210> 10
   <211> 46
   <212> DNA
   <213> Artificial sequence
<220>
   <223> Primer
<400> 10
   tattatacta gttagcgagg attgctgaaa tcttgtatat tttcag 46
<210> 11
   <211> 26
   <212> DNA
   <213> Artificial Sequence
<220>
   <223> Primer
<400> 11
   cccggggcca cgttctaaac taacta 26
<210> 12
   <211> 31
   <212> DNA
   <213> Artificial Sequence
<220>
   <223> Primer
<400> 12
   atggatcctt ttttcttgta cttgtcttct c 31
<210> 13
   <211> 537
   <212> DNA
   <213> Saccharomyces cerevisiae
<220>
   <221> CDS
   <222> (1)..(537)
<400> 13
   gca tcc aac ttg aac att tcg aga aag ctt acc ata tca acc cca tca          48
   Ala Ser Asn Leu Asn Ile Ser Arg Lys Leu Thr Ile Ser Thr Pro Ser
   1               5                   10                  15
   tgc tct ttc gaa aat tca aat agc aca tcc att cct tcg ccc gct tcc          96
   Cys Ser Phe Glu Asn Ser Asn Ser Thr Ser Ile Pro Ser Pro Ala Ser
               20                  25                  30
   tca tct caa agc cac act cca atg aga aac atg agc tca ctc tct gat         144
   Ser Ser Gln Ser His Thr Pro Met Arg Asn Met Ser Ser Leu Ser Asp
           35                  40                  45
   aac agc gtt ttc agc cgg aat atg gaa caa tca tca cca atc act cca         192
   Asn Ser Val Phe Ser Arg Asn Met Glu Gln Ser Ser Pro Ile Thr Pro
       50                  55                  60
   agt atg tac caa ttt ggt cag cag cag tca aac agt ata tgt ggt agc         240
   Ser Met Tyr Gln Phe Gly Gln Gln Gln Ser Asn Ser Ile Cys Gly Ser
   65                  70                  75                  80
   acc gtt agt gtg aat agt ctg gtg aat aca aat aac aaa caa agg atc         288
   Thr Val Ser Val Asn Ser Leu Val Asn Thr Asn Asn Lys Gln Arg Ile
                   85                  90                  95
   tac gaa caa atc acg ggt cct aac agc aat aac gca acc aat gat tat         336
   Tyr Glu Gln Ile Thr Gly Pro Asn Ser Asn Asn Ala Thr Asn Asp Tyr
               100                 105                 110
   att gat ttg cta aac cta aat gag tct aac aag gaa aac caa aat ccc         384
   Ile Asp Leu Leu Asn Leu Asn Glu Ser Asn Lys Glu Asn Gln Asn Pro
           115                 120                 125
   gca acg gcg cat tac ctc aat ggg ggc cca ccc aag aca agc ttc att         432
   Ala Thr Ala His Tyr Leu Asn Gly Gly Pro Pro Lys Thr Ser Phe Ile
       130                 135                 140
   aac cat gga atg ttc ccc tcg cca act ggg acc ata aat agc ggt aaa         480
   Asn His Gly Met Phe Pro Ser Pro Thr Gly Thr Ile Asn Ser Gly Lys
   145                 150                 155                 160
   tct agc agt gcc tca tct tta att tct ttt ggt atg ggc aat acc caa         528
   Ser Ser Ser Ala Ser Ser Leu Ile Ser Phe Gly Met Gly Asn Thr Gln
                   165                 170                 175
   gta ata tag                                                             537
   Val Ile
<210> 14
   <211> 178
   <212> PRT
   <213> Saccharomyces cerevisiae
<400> 14
   Ala Ser Asn Leu Asn Ile Ser Arg Lys Leu Thr Ile Ser Thr Pro Ser
   1               5                   10                  15
   Cys Ser Phe Glu Asn Ser Asn Ser Thr Ser Ile Pro Ser Pro Ala Ser
               20                  25                  30
   Ser Ser Gln Ser His Thr Pro Met Arg Asn Met Ser Ser Leu Ser Asp
           35                  40                  45
   Asn Ser Val Phe Ser Arg Asn Met Glu Gln Ser Ser Pro Ile Thr Pro
       50                  55                  60
   Ser Met Tyr Gln Phe Gly Gln Gln Gln Ser Asn Ser Ile Cys Gly Ser
   65                  70                  75                  80
   Thr Val Ser Val Asn Ser Leu Val Asn Thr Asn Asn Lys Gln Arg Ile
                   85                  90                  95
   Tyr Glu Gln Ile Thr Gly Pro Asn Ser Asn Asn Ala Thr Asn Asp Tyr
               100                 105                 110
   Ile Asp Leu Leu Asn Leu Asn Glu Ser Asn Lys Glu Asn Gln Asn Pro
           115                 120                 125
   Ala Thr Ala His Tyr Leu Asn Gly Gly Pro Pro Lys Thr Ser Phe Ile
       130                 135                 140
   Asn His Gly Met Phe Pro Ser Pro Thr Gly Thr Ile Asn Ser Gly Lys
   145                 150                 155                 160
   Ser Ser Ser Ala Ser Ser Leu Ile Ser Phe Gly Met Gly Asn Thr Gln
                   165                 170                 175
   Val Ile